# EUROPÄISCHE PATENTSCHRIFT

(11) **EP 0 569 611 B1**
(45) Veröffentlichungstag und Bekanntmachung des Hinweises auf die Patenterteilung: **23.07.1997**
(21) Anmeldenummer: 92108101.4
(22) Anmeldetag: 13.05.1992
(51) Int. Cl.: B05B 17/06, A61M 15/00

(54) **Zerstäubungsgerät, insbesondere Taschen-Zerstäubungsgerät**
Spray device, particularly pocket spray device
Pulvérisateur, en particulier pulvérisateur de poche

(43) Veröffentlichungstag der Anmeldung: 18.11.1993
(60) Teilanmeldung: 96112316.3
(73) Patentinhaber: SIEMENS AKTIENGESELLSCHAFT, 80333 München (DE)
(72) Erfinder: Linden, van der, Klaus, W-8630 Coburg (DE) (DE)

(56) Entgegenhaltungen:
- EP-A- 0 111 163
- EP-A- 0 156 409
- DE-A- 3 508 560

## Beschreibung

Die Erfindung bezieht sich auf ein Zerstäubungsgerät, insbesondere Taschen-Zerstäubungsgerät zur Zerstäubung von Arzeimitteln für Inhalationszwecke, mit einem Gehäuse, einem Aufsatz zum Gehäuse, einem Ultraschallzerstäuber, einer elektrischen Stromversorgeeinrichtung, mit einem Vorratsbehälter für eine zu zerstäubende Flüssigkeit, eine Einrichtung zur Beförderung der zu zerstäubenden Flüssigkeit und einem Auslöseelement.

Ein solches Zerstäubungsgerät geht hervor aus der EP 0 111 163 A 2.

In vielen Fällen ist es wünschenswert, eine Flüssigkeit zu zerstäuben. Beispiele hierfür sind im kosmetischen Bereich die Zerstäubung eines Haarlackes und im medizinischen Bereich die Zerstäubung eines Arznei- oder Desinfektionsmittels. Handelsübliche Zerstäubungsgeräte, insbesondere zur Inhalation von Arzneimitteln, wie Sprüh- und Spritzpistolen und mechanische Handvernebler, haben den Nachteil, daß mit diesen keine hinreichend feine Verteilung und Homogenität des Aerosols erreichbar ist. Zudem ist bei den mechanischen Handverneblern ein großer Kraftaufwand erforderlich. Alternativ als Zerstäubungsgeräte einsetzbare Aerosol-Sprühdosen haben insbesondere bei der Anwendung zur Inhalation eines Arzneimittels den Nachteil, daß bei der Applikation ein Kältereiz auftreten kann und die Treibgase eine zum Teil schädliche Wirkung haben. Des weiteren führen die sehr hohen Geschwindigkeiten des Aerosols zu Problemen bei der Koordination der Betätigung der Sprühdose und dem Inhalieren durch den Patienten.

Ein Zerstäubungsgerät mit einer von Hand zu bedienenden Schlauchpumpe zur exakten Dosierung eines Inhalats geht aus der DE 35 08 560 A 1 hervor.

Für die Zerstäubung von Flüssigkeiten sind auch Ultraschallzerstäuber geeignet. Solche Ultraschallzerstäuber, die beispielsweise als Tascheninhalationsgeräte ausgeführt sind, sind aus EP 0 258 637 B 1 und aus der EP 0 373 237 A 1 bekannt.

Diese Zerstäubungsgeräte umfassen ein Gehäuse, in dem unter anderem ein Ultraschallzerstäuber und eine elektrische Stromversorgeeinrichtung angeordnet sind sowie einen Aufsatz zum Gehäuse, in dem ein Vorratsbehälter für eine zu zerstäubende Flüssigkeit angeordnet ist. Zur Betätigung dieser Ultraschallzerstäuber ist eine Krafteinwirkung des Bedieners auf ein Auslöseelement erforderlich, wodurch eine durch die Dimensionierung einer Dosierkammer vorgegebene Flüssigkeitsmenge zu einem Zerstäuberteller geführt wird. Auf dem Zerstäuberteller wird diese Flüssigkeitsmenge zu einem, zum Beispiel lungengängigen, Aerosol zerstäubt. Bekannte Dosiersysteme, die sich als Einrichtung zur Beförderung der Flüssigkeit, zum Beispiel eines Schleppkolben (EP 0 258 637 B1) oder einer Drucktaste (FR 2 444 504 A1) bedienen, haben den Nachteil, daß Fehldosierungen aufgrund der unterschiedlichen Krafteinwirkung des Bedieners auf das Auslöseelement auftreten. Zum anderen kann die Dosiermenge nicht an unterschiedliche zu zerstäubende Flüssigkeiten und deren Medikation angepaßt werden.

Der Erfindung liegt daher die Aufgabe zugrunde, ein Zerstäubungsgerät, insbesondere ein Taschenzerstäubungsgerät, anzugeben, mit dem es möglich ist, Fehldosierungen zu vermeiden und verschiedene vorgebbare Flüssigkeitsmengen zu dosieren.

Diese Aufgabe wird erfindungsgemäß dadurch gelöst, daß die Einrichtung zur Beförderung der Flüssigkeit zum Ultraschallzerstäuber eine Antriebsquelle umfaßt, mittels derer unabhängig von der Einwirkungskraft und -dauer auf das Auslöseelement eine vorgebbare Flüssigkeitsmenge dosierbar ist. Hierdurch werden Fehldosierungen des Bedieners des Zerstäubungsgerätes vermieden, da er keine Kraft zur Beförderung der Flüssigkeit aufbringen muß.

In besonders vorteilhafter Weiterbildung der Erfindung kann die Antriebsquelle die Flüssigkeit mittels Druckeinwirkung befördern. Damit ist das Zerstäubungsgerät lageunabhängig betreibbar.

Zur Entkopplung der Antriebsquelle von einer elektrischen Stromversorgeeinrichtung kann die Flüssigkeit vorteilhafterweise über eine elastische Kraft unter Druck gesetzt werden.

Zweckmäßigerweise kann die Flüssigkeit über eine auf einen Kolben wirkende Federkraft unter Druck gesetzt werden.

Eine besonders zweckmäßige Weiterbildung der Erfindung sieht eine Pumpe in der Antriebsquelle vor. Eine Pumpe eignet sich besonders für die Förderung verschiedener, vorgebbarer Flüssigkeitsmengen, da die Pumpe bezüglich ihrer Drehzahl und der Gesamtzahl der Umdrehungen einfach regelbar ist.

In Weiterbildung der Erfindung kann die Einrichtung zur Beförderung der Flüssigkeit diese bei abgeschaltetem Gerät gegenüber der Umgebungsluft ab dichten. Hierdurch wird ein Austrocknen und/oder Auslaufen der Flüssigkeit bei längerem Nichtgebrauch vermieden und die chemische Reaktion von Umgebungsluft und Arzneimittel unterbunden.

In der Antriebsquelle kann vorteilhafterweise eine Schlauchpumpe verwendet sein. Weil so die zu befördernde Flüssigkeit nur mit dem die Flüssigkeit führenden Schlauch in Berührung kommt, eignet sich eine Schlauchpumpe besonders zur sterilen Beförderung einer Flüssigkeit, insbesondere eines Arzneimittels.

Zur besonders guten Überwachung der zu dosierenden Flüssigkeitsmenge umfaßt die Einrichtung zur Beförderung der Flüssigkeit einen Regler. Dieser ist zugleich Voraussetzung für eine Weiterbildung der Erfindung.

In vorteilhafter Weiterbildung der Erfindung kann dem Regler eine mit dem Vorratsbehälter verknüpfte Codiereinrichtung zugeordnet sein. Mittels der Codiereinrichtung wird dem Regler ein Sollwert vorgegeben, der der zu dosierenden Flüssigkeitsmenge entspricht und auf die im Vorratsbehälter befindliche Flüssigkeit abgestimmt ist.

Es erweist sich als besonders zweckmäßig, wenn der Regler die Einrichtung zur Beförderung der Flüssigkeit abschaltet, sobald die geförderte Flüssigkeitsmenge den Wert erreicht, den die mit dem Vorratsbehälter verknüpfte Codiereinrichtung vorgibt. Hierdurch ist eine genaue Dosierung einer vorgegebenen Flüssigkeitsmenge gewährleistet, da die Angleichung des Ist-Wertes, der mittels der Antriebsquelle geförderten Flüssigkeit an den vorgegebenen Soll-Wert nicht von der Krafteinwirkung des Bedieners abhängt, sondern von dem Regler gesteuert wird.

Ein vorteilhafter Aufbau des Zerstäubungsgeräts wird erreicht, wenn in dem Gehäuse der Ultraschallzerstäuber, die elektrische Stromversorgungseinrichtung und das Auslöseelement eingebaut sind, im Aufsatz zum Gehäuse der Vorratsbehälter und eine mit dem Vorratsbehälter verknüpfte Codiereinrichtung eingebaut sind und die Einrichtung zur Beförderung der zu zerstäubenden Flüssigkeit mit Ausnahme eines im Aufsatz befindlichen, den luftdichten Abschluß der Flüssigkeit bewirkenden Elementes im Gehäuse eingebaut ist. Hierdurch ist es dem Bediener möglich, das Zerstäubungsgerät mit verschiedenen auswechselbaren Aufsätzen zum Gehäuse zu betreiben. Dabei ist die Flüssigkeit in jedem Aufsatz zum Gehäuse gegenüber der Umgebungsluft abgedichtet und für jeden Aufsatz zum Gehäuse eine variable, zu dosierende Flüssigkeitsmenge mittels der zugehörigen Codiereinrichtung vorgegeben.

Ausführungsbeispiele der Erfindung werden anhand der Zeichnung näher erläutert. Dabei zeigen:
Figur 1 einen Längsschnitt durch ein erfindungsgemäßes Zerstäubungsgerät, hier ein Inhalationsgerät für Arzneimittel; und
Figur 2 einen vergrößerten Ausschnitt aus einem anderen erfindungsgemäßen Zerstäubungsgerät.

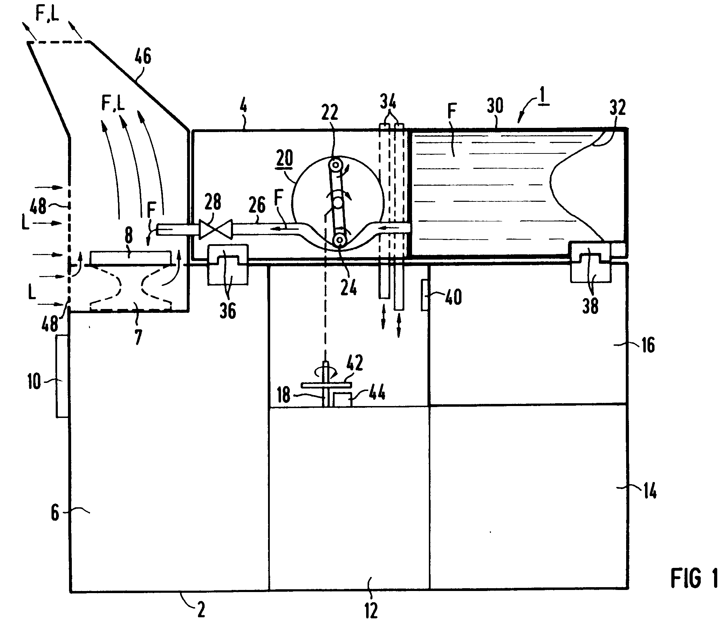

Das in Figur 1 dargestellte Inhalationsgerät 1 umfaßt ein Gehäuse 2 und einen Aufsatz 4 zum Gehäuse 2. Das Gehäuse 2 enthält unter anderem einen Ultraschallzerstäuber 6 mit einem Amplitudentransformator 7, dessen eine Oberfläche als Zerstäuberteller 8 ausgebildet ist, ein Auslöseelement 10, einen Motor 12, eine elektrische Versorgeeinrichtung 14, einen Regler 16 und eine Motorwelle 18. In dem Aufsatz 4 zum Gehäuse 2 ist ein Vorratsbehälter 30 mit der zu zerstäubenden Flüssigkeit F und einem Faltsack 32, eine Codiereinrichtung in Form von Codierstiften 34, eine Schlauchpumpe 20 mit ihren Druckrollen 22, 24, ein Schlauch 26 sowie ein Schlauchventil 28 eingebaut. Der Aufsatz 4 zum Gehäuse 2 ist mittels der Magnethalterungen 36, 38 am Gehäuse 2 auswechselbar befestigt. Außerdem ist im Gehäuse 2 ein Sensor 40 zur Abtastung der Position der Codierstifte 34 vorgesehen. Auf die Motorwelle 18 ist eine Segmentscheibe 42 montiert, deren Drehwinkel mit einem weiteren Sensor 44 abgetastet wird. Bei auf das Gehäuse 2 aufgesetztem Aufsatz 4 koppelt, wie dargestellt, die Motorwelle 18 an die Schlauchpumpe 20 an, der Schlauch 26 ragt in den Raum unmittelbar über dem Zerstäuberteller 8 und die Codierstifte 34 ragen in das Gehäuse 2. Zur Inhalation der zu zerstäubenden Flüssigkeit F sind ein Mundstück 46 und Lufteinlaßöffnungen 48 im Gehäuse 2 vorgesehen.

Nach der Betätigung des Auslöseelementes 10 - im vorliegenden Fall ein kapazitiver Schalter - wertet der Regler 16 mittels des Sensors 40 die Position der Codierstifte 34, mit denen die zu dosierende Flüssigkeitsmenge vorgebbar ist, aus. Unabhängig von der Einwirkungskraft und -dauer auf das Auslöseelement 10 startet der Regler 16 den Motor 12 und den Ultraschallzerstäuber 6 mit seinem Zerstäuberteller 8 und öffnet das Schlauchventil 28. Der Motor 12 treibt über die Motorwelle 18 die Schlauchpumpe 20 an, die mittels der rotierenden Druckrollen 22, 24 die Flüssigkeit F aus dem Vorratsbehälter 30 zum Zerstäuberteller 8 fördert. Die zu dosierende Flüssigkeitsmenge, die mit den Codierstiften 34 vorgegeben wurde, wird mittels der auf der Motorwelle 18 montierten Segmentscheibe 42 und dem Sensor 44 von dem Regler 16 überwacht. Die zu dosierende Flüssigkeitsmenge hängt hierbei von der Anzahl der Umdrehungen der Schlauchpumpe 20, dem Durchmesser des Schlauchs 26 und der Elastizität des Schlauches 26 ab.

Stellt der Regler 16 die Übereinstimmung der von den Codierstiften 34 vorgegebenen Flüssigkeitsmenge mit der von der Schlauchpumpe 20 geförderten Flüssigkeitsmenge fest, schaltet der Regler den Motor 12 ab und schließt das Schlauchventil 28. Die von der Schlauchpumpe 20 geförderte Flüssigkeitsmenge wird auf dem Zerstäuberteller 8 zerstäubt. Saugt der Patient über das Mundstück 46 Luft L durch die Lufteinlaßöffnungen 48 an, wird das sich über dem Zerstäuberteller 8 bildende Aerosol mit der angesaugten Luft L inhaliert. Die mittels der Codierstifte 34 vorgegebene Flüssigkeitsmenge ist an die jeweils im Vorratsbehälter 30 befindliche Flüssigkeit F und deren Dosiermenge angepaßt. Abweichend von dem in Figur 1 dargestellten Ausführungsbeispiel kann die Schlauchpumpe 20 auch so in den Aufsatz 4 zum Gehäuse 2 eingebaut werden, daß ein Normalenvektro einer Ebene, die durch die Drehbewegung der Schlauchpumpe 20 aufgespannt wird, parallel zur Motorwelle 18 orientiert ist. Auf diese Weise kann auf das in Figur 1 nicht weiter dargestellte Getriebe, das in Figur 1 notwendigerweise zwischen die Motorwelle 18 und die Schlauchpumpe 20 geschaltet werden muß, verzichtet werden.

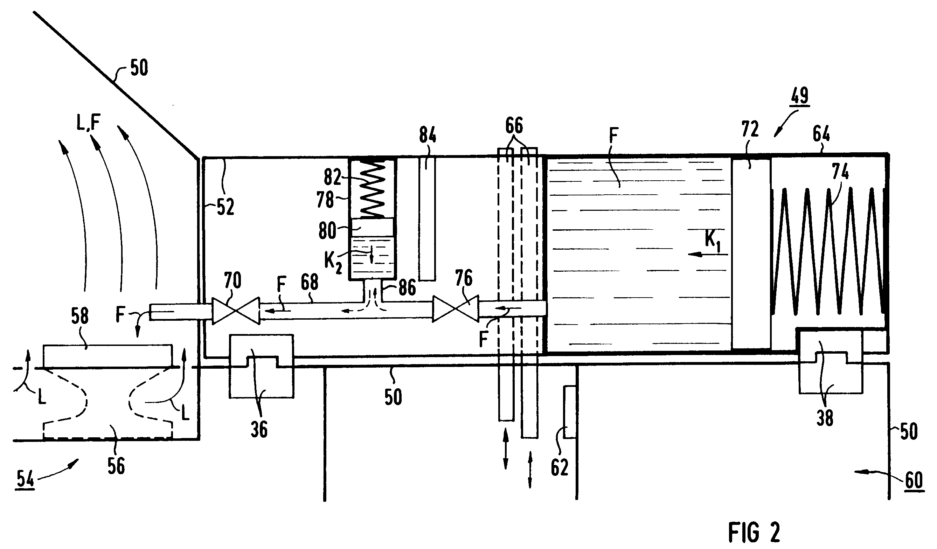

Der in Figur 2 dargestellte Ausschnitt aus einem anderen Zerstäubungsgerät 49 zeigt eine alternative Art zur Beförderung der Flüssigkeit F. Das in der Figur 2 im Ausschnitt dargestellte Zerstäubungsgerät umfaßt - ähnlich wie dies anhand der Figur 1 gezeigt wurde - ein Gehäuse 50 und einen Aufsatz 52 zum Gehäuse 50. Das Gehäuse 50 enthält unter anderem einen Ultraschallzerstäuber 54 mit einem Amplitudentransformator 56, dessen eine Oberfläche als Zerstäuberteller 58 ausgebildet ist, einen Regler 60 und einen Sensor 62. In den Aufsatz 52 zum Gehäuse 50 ist unter anderem ein Vorratsbehälter 64 mit der zu zerstäubenden Flüssigkeit, eine Codiereinrichtung in Form von Codierstiften 66, ein vom Vorratsbehälter 64 zum Zerstäuberteller 58 führender Schlauch 68 mit einem Schlauchventil 70 eingebaut. Abweichend von Figur 1 ist jedoch im Aufsatz 52 ein Kolben 72 im Vorratsbehälter 64, eine mit der Federkraft K₁ auf den Kolben 72 wirkende Feder 74, ein weiteres Schlauchventil 76, ein Zwischenbehälter 78 mit einem Kolben 80 und einer mit der Federkraft K₂ auf den Kolben 80 wirkende Feder 82 und ein Sensor 84 zur Erfassung der Position des Kolbens 80 im Zwischenbehälter 78 eingebaut. Der Zwischenbehälter 78 ist mittels eines T-Stückes 86 zwischen den Schlauchventilen 70 und 76 an den zum Zerstäuberteller 58 führenden Schlauch 68 angeschlossen.

Beim Betrieb des Zerstäubungsgerätes 49 öffnet der Regler 60 nach Betätigung eines Auslöseelementes das ausgangsseitig am Vorratsbehälter 64 angeordnete Schlauchventil 76 bei gleichzeitig geschlossenem Schlauchventil 70. Mittels der Druckeinwirkung der Feder 74, die mit der Kraft K₁ auf den Kolben 72 des Vorratsbehälters 64 wirkt, wird die zu zerstäubende Flüssigkeit F gegen die auf den Kolben 80 wirkende Federkraft K₂ in Zwischenbehälter 78 gedrückt. Die Position des Kolbens 80 in dem Zwischenbehälter 78 wird hierbei von dem Sensor 84 abgetastet und von dem Regler 60 erfaßt. Erreicht die Position des Kolbens 80 in dem Zwischenbehälter 78 ausgehend von der Ausgangsposition die Position, die der mittels der Codierstifte 66 vorgegebenen Flüssigkeitsmenge entspricht, schließt der Regler 60 das am Ausgang des Vorratsbehälters 64 angeordnete Schlauchventil 76. Gleichzeitig öffnet er das dem Zerstäuberteller 58 unmittelbar vorgeschaltete Schlauchventil 70. Die zu dosierende Flüssigkeitsmenge wird mittels der Druckeinwirkung der Feder 82, die mit der Kraft K₂ auf den Kolben 80 des Zwischenbehälters 78 wirkt, zum Zerstäuberteller 58 des Ultraschallzerstäubers 54 gefördert. Nachdem die zu dosierende Flüssigkeitsmenge aus dem Zwischenbehälter 78 befördert ist, schließt der Regler 60 das Ventil 70. Auf diese Weise ist, ebenso wie in dem in Figur 1 gezeigten Fall, unabhängig von der Auslösekraft und -dauer eine vorgebbare Flüssigkeitsmenge beförderbar.

Es wäre auch denkbar, den Sensor 84 am Zwischenbehälter 78 wegzulassen und den Kolben 80 bei jedem Förderhub zwischen zwei Anschlägen im Zwischenbehälter 78 zu verstellen. In diesem Fall wäre nur das Volumen zwischen den beiden Anschlägen dosierbar. Diese Konstruktion zeichnet sich durch einen Preisvorteil aus.

Des weiteren wäre es möglich, ganz auf den Zwischenbehälter 78 und das Schlauchventil 76 zu verzichten und über einen anderen Sensor die Verstellung des Kolbens 72 im Vorratsbehälter 64 abzutasten, um mit den Signalen den Regler 60 anzusteuern und über den Regler 60 das Schlauchventil 76 zu steuern.

## Patentansprüche

1. Zerstäubungsgerät, insbesondere Taschen-Zerstäubungsgerät, mit
a) einem Gehäuse (2),
b) einem Aufsatz (4) zum Gehäuse (2),
c) mit einem Vorratsbehälter (30) für eine zu zerstäubende Flüssigkeit (F),
d) einem Ultraschallzerstäuber (6),
e) einer elektrischen Stromversorgeeinrichtung (14),
f) eine Einrichtung zur Beförderung der zu zerstäubenden Flüssigkeit (F) und
g) einem Auslöseelement (10),
**dadurch gekennzeichnet**, daß die Einrichtung zur Beförderung der Flüssigkeit (F) zum Ultraschallzerstäuber (6) eine Antriebsquelle umfaßt, mittels derer unabhängig von der Einwirkungskraft und -dauer auf das Auslöseelement (10) eine vorgebbare Menge Flüssigkeit (F) dosierbar ist.

2. Zerstäubungsgerät nach Anspruch 1, **dadurch gekennzeichnet**, daß die Antriebsquelle die Flüssigkeit (F) mittels Druckeinwirkung befördert.

3. Zerstäubungsgerät nach Anspruch 1 oder 2, **dadurch gekennzeichnet**, daß die Flüssigkeit (F) über eine elastische Kraft unter Druck setzbar ist.

4. Zerstäubungsgerät nach einem der Ansprüche 1 bis 3, **dadurch gekennzeichnet**, daß die Flüssigkeit (F) über eine auf einen Kolben (72) wirkende Federkraft (K₁) unter Druck setzbar ist.

5. Zerstäubungsgerät nach Anspruch 1 oder 2, **dadurch gekennzeichnet**, daß die Antriebsquelle eine Pumpe umfaßt.

6. Zerstäubungsgerät nach einem der Ansprüche 1, 2 oder 5, **dadurch gekennzeichnet**, daß die Einrichtung zur Beförderung der Flüssigkeit (F) diese bei abgeschaltetem Gerät (1) gegenüber der Umgebungsluft (L) abdichtet.

7. Zerstäubungsgerät nach einem der Ansprüche 1, 2, 3, 5 und 6, **dadurch gekennzeichnet**, daß die Antriebsquelle eine Schlauchpumpe (20) umfaßt.

8. Zerstäubungsgerät nach Anspruch 1, **dadurch gekennzeichnet**, daß die Einrichtung zur Beförderung der Flüssigkeit (F) einen Regler (16, 60) umfaßt, der die zu dosierende Menge Flüssigkeit (F) überwacht.

9. Zerstäubungsgerät nach Anspruch 8, **dadurch gekennzeichnet**, daß dem Regler (16, 60) eine mit dem Vorratsbehälter (30, 64) verknüpfte Codiereinrichtung (34, 66) zugeordnet ist.

10. Zerstäubungsgerät nach Anspruch 8, **dadurch gekennzeichnet**, daß dem Regler (16, 60) eine mit der Einrichtung zur Beförderung der Flüssigkeit (F) verknüpfte Codiereinrichtung (42, 84) zugeordnet ist.

11. Zerstäubungsgerät nach Anspruch 9 oder 10, **dadurch gekennzeichnet**, daß die Codiereinrichtung (34, 66) eine mechanisch abtastbare Struktur ist.

12. Zerstäubungsgerät nach Anspruch 9 oder 10, **dadurch gekennzeichnet**, daß die Codiereinrichtung (34, 66) optisch abtastbar ist.

13. Zerstäubungsgerät nach Anspruch 9 oder 10, **dadurch gekennzeichnet**, daß die Codiereinrichtung (34, 66) elektromagnetisch abtastbar ist.

14. Zerstäubungsgerät nach einem der Ansprüche 8 bis 13, **dadurch gekennzeichnet**, daß der Regler (16, 60) die Einrichtung zur Beförderung der Flüssigkeit (F) abschaltet, sobald die geförderte Flüssigkeitsmenge den Wert erreicht, den die mit dem Vorratsbehälter (30, 64) verknüpfte Codiereinrichtung (34, 66) vorgibt.

15. Zerstäubungsgerät nach einem der Ansprüche 1 bis 14, **dadurch gekennzeichnet**, daß in dem Gehäuse (2) der Ultraschallzerstäuber (6), die elektrische Stromversorgungseinrichtung (14) und das Auslöseelement (10) eingebaut sind, im Aufsatz (4) zum Gehäuse (2) der Vorratsbehälter (30) und eine mit dem Vorratsbehälter (30) verknüpfte Codiereinrichtung (34) eingebaut sind und die Einrichtung zur Beförderung der zu zerstäubenden Flüssigkeit (F) mit Ausnahme eines im Aufsatz befindlichen, den luftdichten Abschluß der Flüssigkeit bewirkenden Elements (22, 24) im Gehäuse (2) eingebaut ist.

## Claims

1. Atomizer, in particular a pocket atomizer, having
a) a housing (2)
b) an attachment (4) for attachment to the housing (2),
c) having a storage container (30) for a fluid (F) which is to be atomized,
d) an ultrasonic atomizer (6),
e) an electric power-supply device (14),
f) a device for conveying the fluid (F) which is to be atomized, and
g) a trigger element (10),
characterised in that the device for conveying the fluid (F) to the ultrasonic atomizer (6) comprises a drive source, by means of which irrespective of the force and duration of action upon the trigger element (10) it is possible to dose a predeterminable quantity of fluid (F).

2. Atomizer according to claim 1, characterised in that the drive source conveys the fluid (F) by means of a pressure action.

3. Atomizer according to claim 1 or 2, characterised in that the fluid (F) can be put under pressure by way of an elastic force.

4. Atomizer according to one of the claims 1 to 3, characterised in that the fluid (F) can be put under pressure by way of a spring force (K₁) which acts on a plunger (72).

5. Atomizer according to claim 1 or 2, characterised in that the drive source comprises a pump.

6. Atomizer according to one of the claims 1, 2 or 5, characterised in that the device for conveying the fluid (F) seals off the latter in respect of the ambient air (L) when the apparatus (1) is switched off.

7. Atomizer according to one of the claims 1, 2, 3, 5 and 6, characterised in that the drive source comprises a hose pump (20).

8. Atomizer according to claim 1, characterised in that the device for conveying the fluid (F) comprises a regulator (16, 60) which monitors the quantity of fluid (F) to be dosed.

9. Atomizer according to claim 8, characterised in that associated with the regulator (16, 60) there is a coding device (34, 66) which is linked to the storage container (30, 64).

10. Atomizer according to claim 8, characterised in that associated with the regulator (16, 60) there is a coding device (42, 84) which is linked to the device for conveying the fluid (F).

11. Atomizer according to claim 9 or 10, characterised in that the coding device (34, 66) is a structure which can be scanned mechanically.

12. Atomizer according to claim 9 or 10, characterised in that the coding device (34, 66) can be scanned visually.

13. Atomizer according to claim 9 or 10, characterised in that the coding device (34, 66) can be scanned electromagnetically.

14. Atomizer according to one of the claims 8 to 13, characterised in that the regulator (16, 60) switches off the device for conveying the fluid (F) as soon as the delivered quantity of fluid attains the value which is predetermined by the coding device (34, 66) which is linked to the storage container (30, 64).

15. Atomizer according to one of the claims 1 to 14, characterised in that the ultrasonic atomizer (6), the electric power-supply device (14) and the trigger element (10) are installed in the housing (2), the storage container (30) and a coding device (34), which is linked to the storage container (30), are installed in the attachment (4) for attachment to the housing (2), and the device for conveying the fluid (F), which is to be atomized, with the exception of an element (22, 24), which is located in the attachment and effects the air-tight seal of the fluid, is installed in the housing (2).

## Revendications

1. Pulvérisateur, et notamment pulvérisateur de poche, comportant :
a) un corps (2),
b) un élément (4) rapporté sur le corps (2),
c) ayant un réservoir (30) pour un liquide (F) à atomiser,
d) un nébuliseur à ultrasons (6),
e) un dispositif d'alimentation en courant électrique (14),
f) un dispositif pour l'acheminement du liquide (F) à atomiser, et
g) un élément de déclenchement (10),
caractérisé par le fait que le dispositif servant à l'acheminement du liquide (F) jusqu'au nébuliseur à ultrasons (6) comprend une source d'entraînement permettant un dosage d'une quantité pouvant être définie à l'avance de liquide (F), indépendamment de la force exercée et de sa durée d'application sur l'élément de déclenchement (10).

2. Vaporisateur selon la revendication 1, caractérisé par le fait que la source d'entraînement achemine le liquide (F) par effet de pression.

3. Vaporisateur selon la revendication 1 ou 2, caractérisé par le fait que le liquide (F) peut être mis sous pression par l'intermédiaire d'une force élastique.

4. Vaporisateur selon l'une des revendications 1 à 3, caractérisé par le fait que le liquide (F) peut être mis sous pression par l'intermédiaire de la force (K₁) d'un ressort, agissant sur un piston (72).

5. Vaporisateur selon la revendication 1 ou 2, caractérisé par le fait que la source d'entraînement comprend une pompe.

6. Vaporisateur selon l'une des revendications 1, 2 ou 5, caractérisé par le fait que le dispositif servant à l'acheminement du liquide (F) le rend étanche vis à vis de l'air ambiant (L), lorsque l'appareil (1) n'est pas utilisé.

7. Vaporisateur selon l'une des revendications 1, 2, 3, 5 et 6, caractérisé par le fait que la source d'entraînement comprend une pompe tubulaire (20).

8. Vaporisateur selon la revendication 1, caractérisé par le fait que le dispositif servant à l'acheminement du liquide (F) comprend un régulateur (16, 60) qui contrôle la quantité de liquide (F) à doser.

9. Vaporisateur selon la revendication 8, caractérisé par le fait qu'un dispositif de codage (34, 66), logiquement relié au réservoir (30, 64), est associé au régulateur (16, 60).

10. Vaporisateur selon la revendication 8, caractérisé par le fait qu'un dispositif de codage (42, 84), logiquement relié au dispositif servant à l'acheminement du liquide (F), est associé au régulateur (16, 60).

11. Vaporisateur selon la revendication 9 ou 10, caractérisé par le fait que le dispositif de codage (34, 66) est une structure pouvant être détectée par voie.

12. Vaporisateur selon la revendication 9 ou 10, caractérisé par le fait que le dispositif de codage (34, 66) peut être lu de manière optique.

13. Vaporisateur selon la revendication 9 ou 10, caractérisé par le fait que le dispositif de codage (34, 66) peut être lu de manière électromagnétique.

14. Vaporisateur selon l'une des revendications 8 à 13, caractérisé par le fait que le régulateur (16, 60) met hors fonction le dispositif servant à l'acheminement du liquide (F), dès que la quantité de liquide acheminée atteint la valeur définie à l'avance par le dispositif de codage (34, 66), logiquement relié au réservoir (30, 64).

15. Vaporisateur selon l'une des revendications 1 à 14, caractérisé par le fait que sont incorporés, dans le corps (2), le nébuliseur à ultrasons (6), le dispositif d'alimentation en courant électrique (14) et l'élément de déclenchement (10), tandis que sont placés, dans l'élément (4) rapporté sur le corps (2), le réservoir (30) et un dispositif de codage (34) logiquement relié au réservoir (30), le dispositif servant à l'acheminement du liquide (F) à atomiser étant incorporé dans le corps (2), à l'exception d'un élément (22, 24) situé dans l'élément rapporté et mettant le liquide à l'abri de l'air.
